# FASCICULE DE BREVET EUROPEEN

(11) **EP 1 603 612 B1**
(45) Date de publication et mention de la délivrance du brevet: **21.10.2009**
(21) Numéro de dépôt: 04719498.0
(22) Date de dépôt: 11.03.2004
(51) Int. Cl.: A61M 5/32

(54) **FOURREAU DE PROTECTION POUR CANULE**
SCHUTZHÜLSE FÜR KANÜLE
PROTECTIVE SHEATH FOR A CANNULA

(30) Priorité: 11.03.2003 FR 0303007
(43) Date de publication de la demande: 14.12.2005
(73) Titulaire: Jouvin, Jean-Luc, 72000 Le Mans (FR)
(72) Inventeur: Jouvin, Jean-Luc, 72000 Le Mans (FR)
(74) Mandataire: Casalonga, Axel
(86) Numéro de dépôt international: PCT/FR2004/000585
(87) Numéro de publication internationale: WO 2004/082730

(56) Documents cités:
- FR-A- 2 716 113
- US-A- 5 201 721
- US-A- 5 554 122
- US-B1- 6 527 742

## Description

La présente invention se rapporte au domaine des dispositifs de protection pour les seringues et notamment pour les seringues à usage dentaire.

La présente invention se rapporte plus particulièrement à un fourreau de protection pour une aiguille d'injection ou canule disposée sur un support porte-canule adaptable sur une seringue, ledit fourreau comportant des moyens de guidage permettant au moins de faire passer ledit porte-canule d'une position de rétraction complète dans laquelle ladite canule est complètement à l'intérieur dudit fourreau à une position d'éjection dans laquelle ladite canule fait saillie au moins partiellement en dehors dudit fourreau et inversement, ledit fourreau comportant également des moyens de blocage final permettant de bloquer définitivement ledit porte-canule dans une position de rétractation après utilisation.

L'art antérieur connaît déjà des fourreaux de protection pour canules disposées sur des supports porte-canule adaptables sur des seringues. Les brevets FR 2 716 113 et US 5 201 721 décrivent des fourreaux de protection.

Il est proposé dans le brevet américain N° US 4 772 272, un manchon de protection pour une seringue jetable ou non jetable.

Le but de cette invention est de simplifier les mouvements du manchon (10) vis-à-vis du support de canule, afin de ne pas nécessiter de rotation, mais uniquement une translation du manchon de protection pour dégager ou couvrir la canule.

Le manchon est muni de premiers moyens permettant de retenir de manière réversible le manchon dans la position aiguille rétractée et de seconds moyens permettant de retenir de manière réversible le manchon dans la position aiguille en érection.

Les premiers moyens sont constitués d'un capuchon supplémentaire, mais cette solution engendre un surcoût à la fabrication et ne présente pas la fiabilité requise. Si le praticien exerce une force trop importante, l'aiguille peut sortir et le piquer.

De plus cette solution ne permet pas de bloquer définitivement le manchon dans une position de protection de la canule après utilisation.

La présente invention entend remédier aux inconvénients de l'art antérieur en proposant un dispositif de protection d'une canule, adaptable sur une seringue non jetable, qui soit simple à fabriquer et à utiliser, peu onéreux, qui permette de fixer la canule sur la seringue sans risque que la canule sorte pendant cette opération et qui présente une position de protection finale dans laquelle il n'est plus possible de faire sortir la canule.

Pour ce faire, la présente invention est du type décrit ci-dessus et elle est remarquable, dans son acception la plus large, en ce que le fourreau comporte en outre des moyens de blocage préalable permettant de bloquer provisoirement ledit porte-canule dans une position de rétractation préalablement à l'utilisation.

Le fourreau comporte, de préférence :
- des moyens de transition primaire permettant d'amener le porte-canule dans la position de rétractation préalablement à l'utilisation.
- des moyens de transition secondaire permettant d'amener le porte-canule de la position de rétractation préalablement à l'utilisation à une position de rétractation en utilisation.
- des moyens de transition tertiaire permettant d'amener le porte-canule d'une position de rétractation en utilisation à une position de rétractation après utilisation.

Lesdits moyens de transition primaires, secondaires et tertiaires sont, de préférence, à transition unique, c'est-à-dire qu'il(s) ne permet(tent) qu'un passage unique d'une position particulière à une autre position particulière.

Lesdits moyens de transition primaires et/ou lesdits moyens de transition secondaires et/ou lesdits moyens de transition tertiaires sont constitués, de préférence, chacun au moins d'une partie de transition présentant, en coupe longitudinale un évidemment réalisé dans la face intérieure dudit fourreau, ledit ou lesdits moyen(s) de transition présentant à chacune de ses ou de leurs extrémités un trou et entre ces trous un pan incliné conduisant progressivement la profondeur p dudit évidemment à une valeur nulle, voire négative.

Ladite partie de transition comporte, de préférence, en outre en coupe longitudinale, entre le pan incliné et le trou, un pan droit.

Dans une variante, le trou situé à proximité de la profondeur p nulle, voire négative, est prolongé au moins partiellement, de chaque côté du plan incliné par des fentes longitudinales.

Dans une autre variante, lesdits moyens de transition primaires et/ou secondaires et/ou tertiaires sont constitués chacun d'une partie de transition constituée d'un ergot, cet ergot étant orienté de préférence radialement.

Le fourreau comporte, de préférence, à son extrémité proximale des moyens d'accueil permettant de positionner ledit porte-canule dans une position d'introduction.

Les moyens d'accueil et/ou lesdits moyens de guidage sont, de préférence, constitués au moins d'une partie fendue longitudinalement et débouchant au moins sur la face interne dudit fourreau.

Lesdits moyens de transition primaires et/ou lesdits moyens de transition secondaires et/ou lesdits moyens de transition tertiaires et/ou lesdits moyens de guidage sont, de préférence, constitués de deux parties identiques positionnées diamétralement opposées selon un axe dudit fourreau.

Lesdits moyens de blocage préalable sont, de préférence, constitués par un ergot, cet ergot étant orienté de préférence radialement.

Dans une version préférée, ledit fourreau comporte un taquet s'étendant vers l'intérieur dudit fourreau et ledit porte-canule comporte au moins une fente longitudinale primaire et au moins une fente longitudinale secondaire destinées à coopérer avec ledit taquet.

La présente invention se rapporte également à un ensemble d'injection constitué par un fourreau selon l'invention, dans lequel est introduite une aiguille d'injection ou canule disposée sur un support porte-canule.

Ledit porte-canule présente, de préférence, au moins deux bossages longitudinaux positionnés diamétralement opposés par rapport à l'axe dudit porte-canule.

La présente invention se rapporte également à une seringue comportant une aiguille d'injection ou canule, ladite seringue étant équipée d'un fourreau selon l'invention.

Avantageusement, la présente invention présente une position, la position de rétraction préalable à l'utilisation, qui permet de clipser, ou éventuellement de visser, en toute sécurité l'extrémité distale de la seringue sur le porte-canule, sans risquer l'éjection de la canule pendant cette opération.

Lorsque l'ensemble d'injection est commercialisé en position de rétraction préalable à l'utilisation, la canule (2) ne peut sortir accidentellement du fourreau vers l'extrémité proximale ou vers l'extrémité distale du fourreau car les moyens de blocage préalable l'en empêchent.

Pour pouvoir utiliser la canule en tant qu'aiguille, il faut volontairement retirer le porte-canule vers l'extrémité proximale du fourreau, puis pousser le porte-canule vers l'extrémité distale du fourreau pour activer les moyens de transition secondaires. Le porte-canule arrive alors en position de rétractation en utilisation et peut être poussé dans les moyens de guidage pour faire sortir la canule. Tant que le moyen de transition tertiaire n'est pas franchi, la canule peut être rentrée dans le fourreau ou sortie du fourreau autant de fois que souhaité.

Avantageusement également, la présente invention présente une position, la position de rétractation après utilisation, dans laquelle il n'est plus possible de provoquer l'éjection de la canule. Dans cette position, la paroi du fourreau s'oppose à ce que le porte-canule avance vers l'extrémité distale du fourreau et des fentes ménagées latéralement dans la zone femelle du filetage qui permet de solidariser le porte-canule avec l'extrémité distale de la seringue provoquent la désolidarisation du porte-canule et de la seringue lorsque l'on tire le porte-canule vers l'extrémité proximale du fourreau.

Dans cette position de rétractation après utilisation, la canule ne peut donc pas être réutilisée.

On comprendra mieux l'invention à l'aide de la description, faite ci-après à titre purement explicatif, d'un mode de réalisation de l'invention, en référence aux figures annexées :
- la figure 1 illustre une vue de face d'un ensemble d'injection selon l'invention, composé d'un fourreau, d'une canule et d'un porte-canule ;
- la figure 2 illustre une vue de face d'un porte canule ;
- la figure 3 illustre une vue de face du porte canule de la figure 2 tourné de 90° par rapport à son axe longitudinal ;
- la figure 4 illustre une vue en coupe longitudinale selon AA' de la figure 3 ;
- la figure 5 illustre une vue de dessus d'un porte-canule ;
- la figure 6 illustre une vue de dessous d'un porte-canule ;
- la figure 7 illustre l'organigramme des différentes positions du porte-canule vis-à-vis du fourreau ;
- la figure 8 illustre une vue de face du fourreau et des différentes positions des bossages du porte-canule ;
- la figure 9 illustre une vue en coupe longitudinale selon CC' de la figure 8 ;
- la figure 10 illustre une vue en coupe longitudinale selon DD' de la figure 8 ;
- la figure 11 illustre une vue en coupe longitudinale selon FF' de la figure 8 ;
- la figure 12 est une vue de face partielle et agrandie d'une variante de réalisation du moyen de transition tertiaire ;
- la figure 13 est une vue en coupe selon GG' de la figure 12 ;
- la figure 14 illustre une vue de face du porte canule pour la variante de la figure 12 ; et
- la figure 15 illustre une vue de dessus d'un porte-canule de la figure 14.

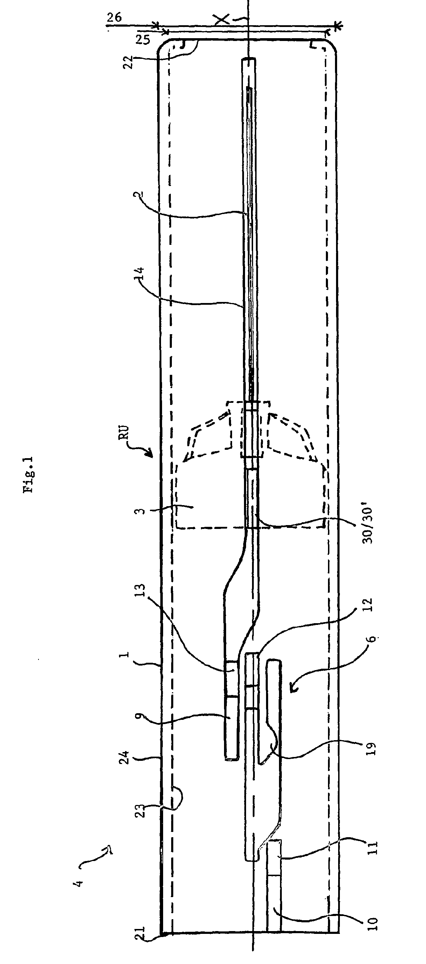

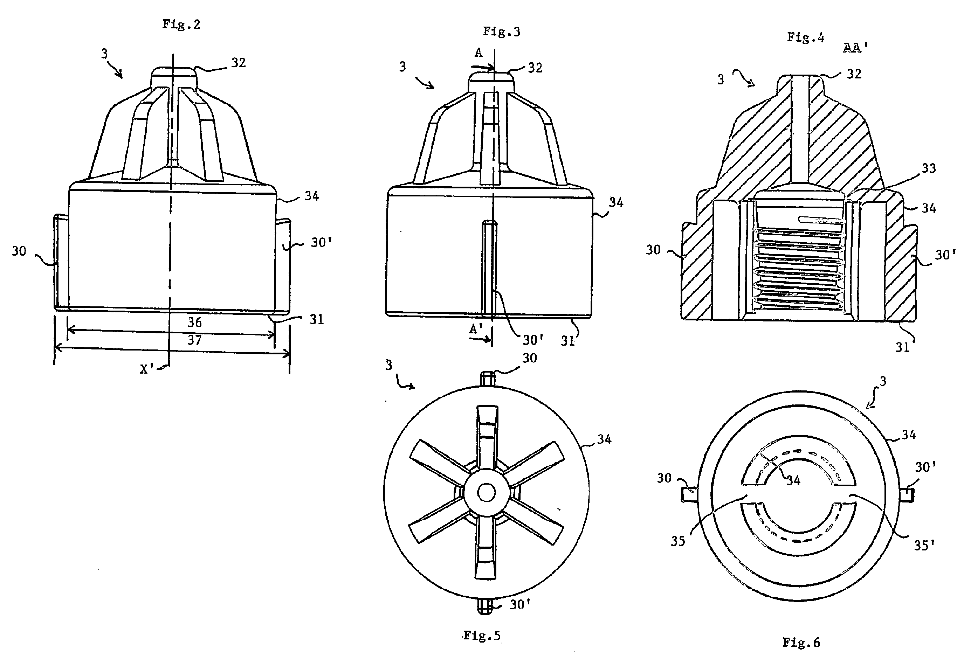

Une seringue dentaire pour l'injection, par exemple, d'un anesthésiant local peut être équipée d'un fourreau (1), illustré figure 1, protégeant une aiguille d'injection ou canule (2), disposée sur un support porte-canule (3), illustré figures 2 à 6. Le fourreau (1), la canule (2) et le porte-canule (3) forment ainsi un ensemble d'injection (4).

L'ensemble d'injection (4) selon l'invention n'est constitué que du fourreau (1) et du porte-canule (3) supportant la canule (2). Aucune autre pièce que le fourreau (1) n'est prévue pour la protection de la canule (2).

Le fourreau (1) présente une forme générale de tube cylindrique présentant un axe longitudinal X. Le fourreau est ouvert à ses extrémités proximale (21) et distale (22). Il est réalisé, par exemple en matière plastique transparente et présente une épaisseur sensiblement constante sur toute sa longueur. Toutefois, pour économiser de la matière, il est possible de réduire l'épaisseur du fourreau (1) dans les parties qui ne comportent pas de moyens de protection particuliers, c'est-à-dire, dans les parties hautes et basses au regard de la figure 1.

Le fourreau (1) présente une face intérieure (23) et une face extérieure (24) par rapport à l'axe X. Il présente également un diamètre intérieur (25) et un diamètre extérieur (26).

Le porte-canule (3) est destiné à être positionné provisoirement à l'extrémité distale de la seringue. Il assure la communication du fluide entre la carpule contenant le liquide à injecter et la canule (2).

Le porte-canule (3) présente une forme générale de tube cylindrique présentant un axe longitudinal X'. Le porte-canule (3) est ouvert à son extrémité proximale (31) et est refermé à son extrémité distale (32) sur la canule (2) ou sur une cavité cylindrique pour l'accueil de la canule, comme visible sur la figure 4. Il présente également une face intérieure (33) et une face extérieure (34) par rapport à l'axe X'.

La face intérieure (33) est de préférence munie d'un filetage, afin de permettre de clipser, voire de visser, le porte-canule (3) sur la seringue. Ce filetage est de préférence interrompu par deux fentes longitudinales (35, 35'), comme on peut le voir sur la figure 6, pour permettre le clipsage par écartement de deux parties filetées formant chacune une mâchoire, lors du positionnement du porte-canule sur le filetage de l'extrémité distale de la seringue.

Le porte-canule (3) présente également un diamètre extérieur (36), légèrement inférieur au diamètre intérieur (25) du fourreau (1).

Le porte-canule (3) comporte en outre au moins un et de préférence deux bossages (30, 30') positionnés diamétralement opposés par rapport à l'axe X'. Au niveau de ces bossages, le porte-canule (3) présente une largeur hors tout (37) supérieure au diamètre intérieur (25) du fourreau (1), et sensiblement identique, voire légèrement inférieure, au diamètre extérieur (26) du fourreau (1).

L'extrémité distale des bossages (30, 30') n'est, de préférence, pas perpendiculaire à l'axe X', mais inclinée vers l'extrémité proximale du porte-canule (3).

Le porte-canule (3) est destiné à être introduit par l'extrémité proximale (21) du fourreau (1), de manière à ce que les axes X et X', respectivement du fourreau et du porte-canule (3) coïncident.

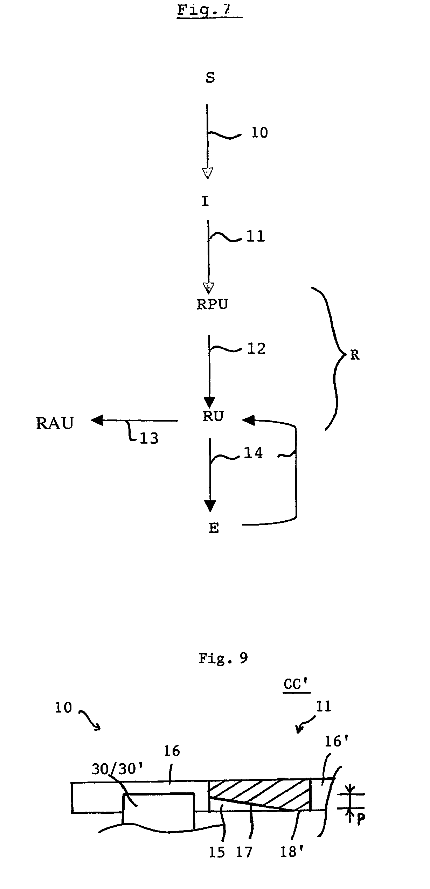

L'ensemble d'injection (4) formé d'une part par la canule (2) et le ledit porte-canule (3) et d'autre part par le fourreau (1), présente une pluralité de positions, illustrées schématiquement sur la figure 7 :
- une position séparée S, dans laquelle d'une part le porte-canule (3) et la canule (2) et d'autre part le fourreau (1) sont complètement séparés ;
- une position d'introduction I, dans laquelle le porte-canule (3) est juste introduit dans l'extrémité proximale du fourreau (1), la canule (2) étant, bien sûr, dans le fourreau (1) et les bossages (30, 30') du porte-canule (3) étant positionnés dans les rainures des moyens d'accueil (10) ;
- une position de rétraction complète R dans laquelle le porte-canule (3) et la canule (2) sont complètement à l'intérieur dudit fourreau (1) ; cette position présentant trois variantes :
   1- une position de rétractation préalablement à l'utilisation RPU, dans laquelle le porte-canule (3) et la canule (2) sont bloqués provisoirement par des moyens de blocage préalable (6) ; Dans cette position, le porte-canule (3) est en butée longitudinale distale, c'est-à-dire que les ergots (30, 30'), en butant contre la paroi du fourreau (1), empêchent le porte-canule (3) d'aller plus vers l'extrémité distale du fourreau (1) ;
   2- une position de rétractation en utilisation RU, dans laquelle le porte-canule (3) et la canule (2) sont à l'intérieur du fourreau (1), mais le porte-canule (3) n'est pas bloqué longitudinalement ;
   3- une position de rétractation après utilisation RAU, dans laquelle le porte-canule (3) et la canule (2) sont à l'intérieur du fourreau (1), bloqués définitivement par des moyens de blocage final (9) ; dans cette position, le porte-canule (3) ne peut plus être bougé par rapport au fourreau (1), ni vers l'avant, ni vers l'arrière ;
- une position d'éjection E dans laquelle le porte-canule (3) est sensiblement à l'extrémité distale du fourreau (1) et dans laquelle la canule (2) fait saillie sensiblement complètement en dehors dudit fourreau (1). Cette position d'éjection E n'est possible qu'après passage en position de rétractation en utilisation RU et n'est plus possible après passage en position de rétractation après utilisation RAU.

Il est important de constater que les positions S et I sont importantes pour « activer » l'ensemble d'injection selon l'invention, c'est-à-dire pour le préparer afin qu'il puisse être utilisé, mais que ces positions ne sont, de préférence, pas visibles par l'utilisateur final. En effet, l'ensemble d'injection est de préférence directement commercialisé avec le porte-canule (3) et la canule (2) en position de rétractation préalablement à l'utilisation RPU.

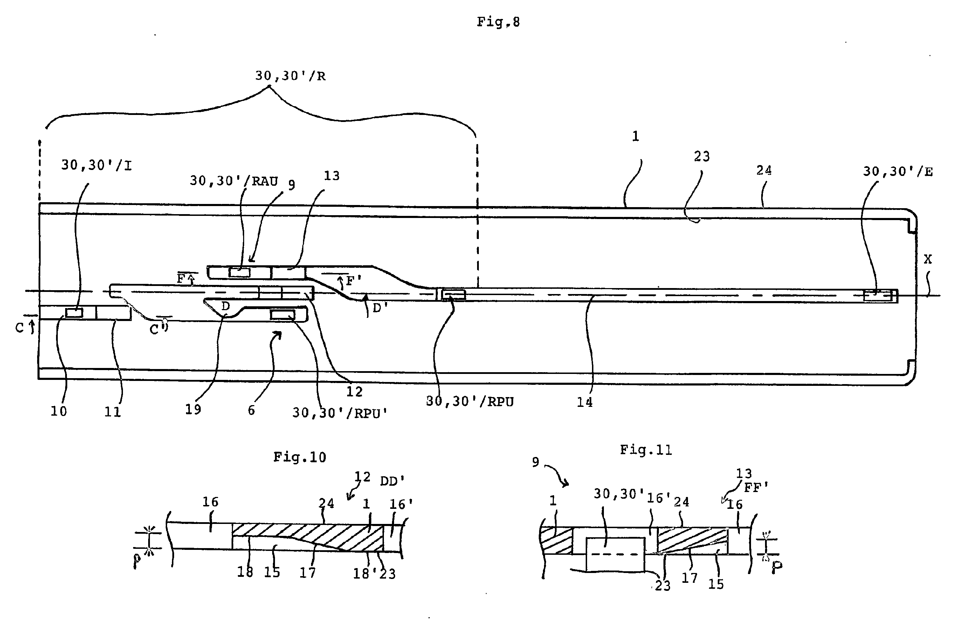

Pour réaliser le passage d'une position à l'autre, le fourreau (1) comporte, comme illustré figure 8 :
- des moyens de transition primaires (11) pour faire passer le porte-canule et la canule de la position d'introduction I à la position de rétractation préalablement à l'utilisation RPU ;
- des moyens de transition secondaires (12) pour faire passer le porte-canule et la canule de la position de rétractation préalablement à l'utilisation RPU à la position de rétractation en utilisation RU ;
- des moyens de guidage (14) pour faire passer le porte-canule et la canule de la position de rétractation en utilisation RU à la position d'éjection E, et inversement, autant de fois que souhaité ;
- des moyens de transition tertiaires (13) pour faire passer le porte-canule et la canule de la position de rétractation en utilisation RU à la position de rétractation après utilisation RAU ;
- des moyens de blocage final (9) permettant de bloquer définitivement ledit porte-canule (3) dans une position de rétractation après utilisation RAU.

Les moyens de guidage (14) sont constitués, par exemple de deux fentes longitudinales parallèles entre elles et à l'axe X, ces deux fentes débouchant de préférence sur la face intérieure (23) du fourreau (1), mais pas sur la face extérieure (24), afin d'obtenir un renfort grâce à la matière qui est dans le fond des fentes et former ainsi des rainures ; toutefois, il est possible de réaliser les fentes débouchant à la fois sur les faces intérieure (23) et extérieure (24) du fourreau (1).

Le fourreau (1) comporte en outre une butée distale, positionnée à l'extrémité distale des moyens de guidage (14). Cette butée est formée par l'extrémité distale de la fente des moyens de guidage (14) et/ou par le retour de la paroi du fourreau vers l'intérieur du fourreau à son extrémité distale.

Les moyens de transition primaires (11), secondaires (12) et tertiaires (13) sont, de préférence, à transition unique, c'est-à-dire qu'ils n'autorisent qu'une seule transition ou un seul passage d'une position préalable vers une position ultérieure et n'autorisent pas la transition ou le passage dans l'autre sens c'est-à-dire le retour à la position préalable.

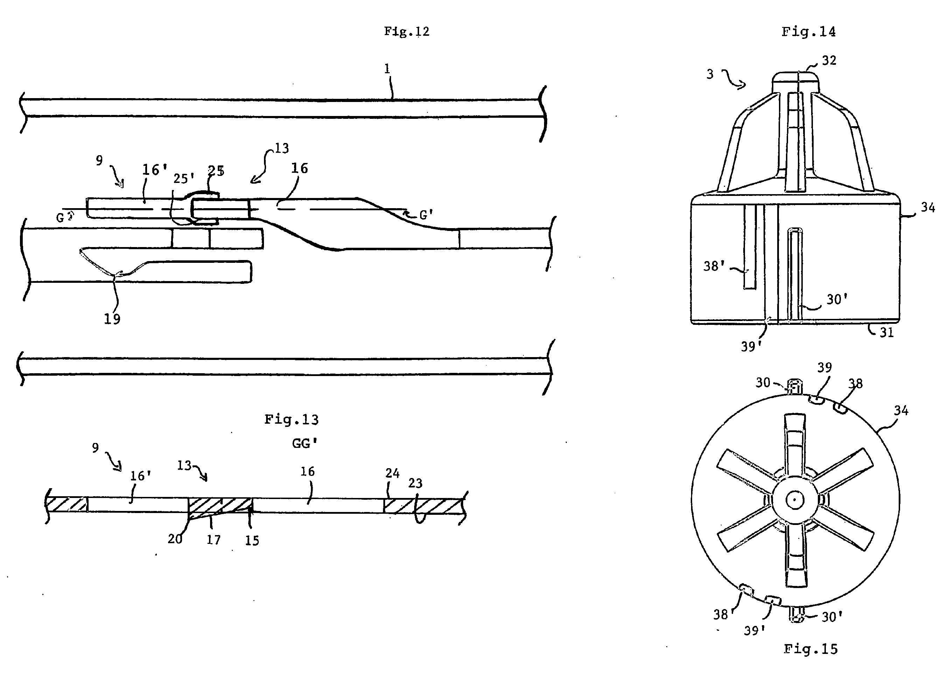

Dans l'exemple illustré figures 8 à 11, ces moyens de transition primaires (11), secondaires (12) et tertiaires (13) sont constitués chacun, de chaque côté par rapport à l'axe X, d'une partie de transition présentant, en coupe longitudinale :
- un évidemment (15) réalisé dans la face intérieure (23) dudit fourreau (1) et ne débouchant que sur la face intérieure (23) du fourreau (1),
- ledit moyen de transition présentant à chacune de ses extrémités un trou (16, 16') débouchant sur les faces intérieure (23) et extérieure (24) du fourreau (1) et
- entre ces trous (16, 16') un pan (17) incliné vers l'intérieur, conduisant progressivement la profondeur p dudit évidemment (15) à une valeur nulle.
   Dans une variante, illustrée figure 9 pour le moyen de transition primaire (11), ladite partie de transition comporte en outre en coupe longitudinale, entre le pan incliné (17) et le trou (16'), un pan droit (18'), sensiblement parallèle à l'axe X.
   Dans une variante, illustrée figure 10 pour le moyen de transition secondaire (12), ladite partie de transition comporte en outre en coupe longitudinale, d'une part entre le trou (16) et le pan incliné (17) et d'autre part entre le pan incliné (17) et le trou (16'), respectivement deux pans droits (18, 18'), sensiblement parallèle à l'axe X.
   Dans la variante de base illustrée figure 11, la partie de transition du moyen de transition comporte uniquement entre les trous (16, 16') un pan (17) incliné vers l'intérieur.
   Dans toutes les variantes, les pans inclinés (17) présentent une longueur de l'ordre de 5 mm pour une profondeur p de l'ordre de 1 mm, c'est-à-dire une pente de l'ordre de 20 % ± 5 %. Une pente douce favorise le passage en douceur d'une position à une autre.
   Chacune des variantes illustrée figures 9, 10 ou 11 est applicable à l'une ou l'autre des parties de transition des moyens de transition primaires (11), secondaires (12) ou tertiaires (13).
   Dans une variante illustrée figures 13 et 14, la partie de transition du moyen de transition tertiaires (13) comporte entre les trous (16, 16') un pan (17) incliné vers l'intérieur du fourreau, afin de former un taquet (20) et le trou (16') est prolongé, de chaque côté du plan incliné (17) et sur environ un tiers de sa longueur, par des fentes longitudinales (25, 25'). Les fentes longitudinales (25, 25') débouchent à la fois sur les faces intérieure (23) et extérieure (24) du fourreau (1), afin de donner encore plus de souplesse au passage final, tout en offrant une résistance plus grande au retour en position RU. Dans cette variante, la profondeur p de l'évidemment (15) atteint ainsi en quelque sorte une valeur négative.
   Ce taquet (20) permet de s'opposer d'une manière plus forte au retour à la position précédente, c'est-à-dire à la position de rétractation en utilisation. Toutefois, pour ne pas gêner le mouvement du porte-canule (3), ce dernier est alors muni
- de deux fentes longitudinales primaires (38, 38') ne s'étendant pas sur toute la longueur du porte-canule de manière à former un blocage supplémentaire en position de rétractation préalablement à l'utilisation RPU, venant s'ajouter aux moyens de blocage préalable (6) ; et
- de deux fentes longitudinales secondaires (39, 39') s'étendant sur la longueur du porte-canule de manière à ce que le taquet (20) ne gêne pas le franchissement des moyens de transition secondaire (12).

Ces deux paires de fentes (38, 39), (38', 39'), visibles sur les figures 14 et 15, sont ménagées sur la surface extérieure (34) du porte-canule (3), à proximité respectivement des bossages (30, 30'). Elles présentent une profondeur au moins égale, voire supérieure à l'épaisseur du taquet (20) vis-à-vis de la paroi intérieure (23) du fourreau (1).

Dans la version illustrée, lesdits moyens de blocage préalable (6) sont constitués par un ergot (19) qui est positionné entre deux trous débouchant chacun sur les faces intérieure (23) et extérieure (24) du fourreau (1).

Cet ergot est configuré de manière à faciliter le maintien des bossages (30, 30') dans la position de rétractation préalable à l'utilisation RPU, c'est-à-dire de manière à faciliter le passage vers la position de rétractation préalable à l'utilisation RPU et à s'opposer au passage de la position de rétractation préalable à l'utilisation RPU vers la position de rétractation en utilisation RU. Pour ce faire, l'ergot (19) présente à son extrémité un pan incliné proximal et un appendice distal.

Il est également possible d'imaginer que lesdits moyens de transition primaires (11) et/ou secondaires (12) et/ou tertiaires (13) soient constitués chacun d'une partie de transition constituée d'un ergot identique à l'ergot (19). Le pan incliné est alors positionné du côté où l'on souhaite faciliter le mouvement de transition et l'appendice est alors positionné du côté où l'on souhaite s'opposer à un mouvement de transition.

Dans la version illustrée, lesdits moyens de blocage final (9) sont constitués par deux trous diamétralement opposés débouchant sur les faces intérieure (23) et extérieure (24) du fourreau (1).

Le fourreau (1) selon l'invention comporte en outre, de préférence, à son extrémité proximale des moyens d'accueil (10) permettant de positionner ledit porte-canule (3) dans une position d'introduction I. Ces moyens d'accueil (10) sont constitués, de préférence, de trous (16), diamétralement opposés par rapport à l'axe X, débouchant au moins sur la face intérieure (23) du fourreau (1), comme on peut le voir sur la figure 9.

Dans le cas où l'ensemble d'injection selon l'invention est commercialisé en position de rétractation préalablement à l'utilisation RPU, c'est donc en usine que les moyens de transition primaires (11) seront mis en oeuvre, manuellement ou grâce à un automatisme, pour faire passer le porte-canule et la canule de la position d'introduction I à la position de rétractation préalablement à l'utilisation RPU, en poussant le porte-canule muni de sa canule dans le fourreau tout en maintenant fermement le fourreau.

L'utilisation du fourreau (1) va maintenant être décrite en détail en partant de la position de commercialisation, la position de rétractation préalablement à l'utilisation RPU. Dans cette position, l'extrémités distale de la seringue peut être clipsée, ou éventuellement vissée, dans le porte-canule (3), grâce au filetage ménagé sur la face intérieure (33) du porte-canule (3), car la paroi distale du trou dans lequel les bossages se trouvent les retient longitudinalement.

Grâce à l'élasticité de l'ergot (19), lorsque les bossages (30, 30') sont dans la position de rétractation préalable à l'utilisation RPU, il suffit de tirer la seringue vers l'arrière - c'est-à-dire vers l'extrémité proximale du fourreau (1) - pour que les bossages (30, 30') poussent transversalement l'ergot et viennent dans le trou (16', 16) commun, respectivement aux moyens de transition primaires (11) et secondaires (12).

Dans ce trou, les bossages (30, 30') ne peuvent plus revenir en position d'introduction, car la paroi transversale du fourreau adjacente au pan (18'), visible figure 9, les en empêche.

La mise en oeuvre des moyens de transition secondaires (12) pour faire passer le porte-canule et la canule de la position de rétractation préalablement à l'utilisation RPU à la position de rétractation en utilisation RU est opérée en poussant la seringue d'une main dans le fourreau tout en maintenant fermement le fourreau (1) de l'autre main.

Les moyens de guidage (14) autorisent le passage de la position de rétractation en utilisation RU à la position d'éjection E en poussant la seringue d'une main tout en maintenant fermement le fourreau (1) de l'autre main, le passage de la position d'éjection E à la position de rétractation en utilisation RU en tirant sur la seringue d'une main tout en maintenant fermement le fourreau (1) de l'autre malin. Toutes les positions intermédiaires sont, bien sûr possibles. Il est évident que l'utilisateur doit laisser l'espace situé devant l'extrémité distale du fourreau libre lorsqu'il utilise les moyens de guidage (14), afin de ne pas piquer accidentellement quelqu'un ou lui-même.

La mise en oeuvre des moyens de transition tertiaires (13) pour faire passer le porte-canule et la canule de la position de rétractation en utilisation. RU à la position de rétractation après utilisation RAU est opérée en tirant sur la seringue d'une main tout en maintenant fermement le fourreau (1) de l'autre main.

Le porte-canule (3) est alors bloqué définitivement par les moyens de blocage final (9).

Pour séparer la seringue de l'ensemble d'injection, il suffit alors de tirer fortement sur la seringue d'une main tout en maintenant fermement le fourreau (1) de l'autre main, afin de désengager le porte-canule (3) de l'extrémité distale de la seringue. Lors de ce mouvement de traction, les deux parties du filetage de la face intérieure (33) du porte-canule (3) séparées par les fentes longitudinales (35, 35') s'écartent afin de libérer l'extrémité distale de la seringue.

Après utilisation, le porte-canule (3) reste donc bloqué en position de rétractation après utilisation RAU dans le fourreau (1), la canule (2) étant protégée par le fourreau (1) ; il n'est plus possible de faire sortir la canule (2) par une des extrémités du fourreau, même en introduisant à nouveau la seringue dans le fourreau et en tentant de clipser ou de visser à nouveau l'extrémité distale de la seringue sur le porte-canule (3), car les moyens de transition tertiaire (13) s'opposent à tout mouvement du porte-canule (3).

L'invention est décrite dans ce qui précède à titre d'exemple. Il est entendu que l'homme du métier est à même de réaliser différentes variantes de l'invention sans pour autant sortir du cadre du brevet.

## Revendications

1. Fourreau (1) de protection pour une aiguille d'injection ou canule (2) disposée sur un support porte-canule (3) adaptable sur une seringue, ledit fourreau comportant des moyens de guidage (14) permettant au moins de faire passer ledit porte-canule (3) d'une position de rétraction complète R dans laquelle ladite canule (2) est complètement à l'intérieur dudit fourreau (1) à une position d'éjection E dans laquelle ladite canule (2) fait saillie au moins partiellement en dehors dudit fourreau (1) et inversement, ledit fourreau (1) comportant également des moyens de blocage final (9) permettant de bloquer définitivement ledit porte-canule (3) dans une position de rétractation après utilisation RAU et comportant des moyens de transition secondaires (12), actionnés par un déplacement longitudinal du porte-canule (3) et permettant le passage du porte-canule (3) d'une position de rétractation préalablement à l'utilisation RPU à une position de rétractation en utilisation RU, des moyens de blocage préalable (6) permettant de bloquer provisoirement ledit porte-canule (3) dans la position de rétractation préalablement à l'utilisation RPU, **caractérisé en ce qu'**il comporte des moyens de blocage préalables (6) comprenant une paroi distale d'un trou destiné à recevoir des bossages du porte-canule (3), la paroi étant apte à retenir longitudinalement les bossages du porte-canule (3) de manière qu'une extrémité distale de la seringue soit clipsable dans le porte-canule.

2. Fourreau (1) de protection selon la revendication 1, **caractérisé en ce qu'**il comporte des moyens de transition primaire (11) permettant d'amener le porte-canule (3) dans la position de rétractation préalablement à l'utilisation RPU.

3. Fourreau (1) de protection selon la revendication 1 ou 2, **caractérisé en ce qu'**il présente une extrémité proximale (21) apte à recevoir la seringue et que les moyens de blocage (6) comportent une butée longitudinale distale.

4. Fourreau (1) de protection selon l'une quelconque des revendications 1 à 3, **caractérisé en ce qu'**il comporte des moyens de transition tertiaire (13) permettant d'amener le portc-canule (3) d'une position de rétractation en utilisation RU à une position de rétractation après utilisation RAU.

5. Fourreau (1) de protection selon l'une quelconque des revendications 2 à 4, **caractérisé en ce que** lesdits moyens de transition primaires (11), secondaires (12) et tertiaires (13) sont à transition unique.

6. Fourreau (1) de protection selon l'une quelconque des revendications 2 à 5, **caractérisé en ce que** lesdits moyens de transition primaires (II) et/ou lesdits moyens de transition secondaires (12) et/ou lesdits moyens de transition tertiaires (13) sont constitués chacun au moins d'une partie de transition présentant, en coupe longitudinale un évidemment (15) réalisé dans la face intérieure dudit fourreau (1), ledit ou lesdits moyen(s) de transition présentant à chacune de ses ou de leurs extrémités un trou (16, 16') et entre ces trous (16, 16') un pan incliné (17) conduisant progressivement la profondeur p dudit évidemment (15) à une valeur nulle, voire négative.

7. Fourreau (1) de protection selon la revendication 6, **caractérisé en ce que** ladite partie de transition comporte en outre en coupe longitudinale, entre le pan incliné (17) et le trou (16, 16'), un pan droit (18, 18').

8. Fourreau (1) de protection selon la revendication 7, **caractérisé en ce que** ledit trou (16, 16') situé à proximité de la profondeur p nulle, voire négative, est prolongé au moins partiellement, de chaque côté du plan incliné (17) par des fentes longitudinales (23, 23').

9. Fourreau (1) de protection selon l'une quelconque des revendications 2 à 5, **caractérisé en ce que** lesdits moyens de transition primaires (11) et/ou secondaires (12) et/ou tertiaires (13) sont constitués chacun d'une partie de transition constituée d'un ergot.

10. Fourreau (1) de protection selon l'une quelconque des revendications 1 à 9, **caractérisé en ce qu'**il comporte à son extrémité proximale des moyens d'accueil (10) permettant de positionner ledit porte-canule (3) dans une position d'introduction I.

11. Fourreau (1) de protection selon l'une quelconque des revendications 1 à 10, **caractérisé en ce que** lesdits moyens d'accueil (10) et/ou lesdits moyens de guidage (14) sont constitués au moins d'une partie fendue longitudinalement et débouchant au moins sur la face interne (23) dudit fourreau (1).

12. Fourreau (1) de protection selon l'une quelconque des revendications 2 à 11, **caractérisé en ce que** lesdits moyens de transition primaires (11) et/ou lesdits moyens de transition secondaires (12) et/ou lesdits moyens de transition tertiaires (13) et/ou lesdits moyens de guidage (14) sont constitués de deux parties identiques positionnées diamétralement opposées selon un axe X dudit fourreau (1).

13. Fourreau (1) de protection selon l'une quelconque des revendications 1 à 12, **caractérisé en ce que** lesdits moyens de blocage préalable (6) sont constitués par un ergot (19).

14. Fourreau (1) de protection selon l'une quelconque des revendications 1 à 13, **caractérisé en ce qu'**il comporte un taquet (20) s'étendant vers l'intérieur dudit fourreau (1) et **en ce que** ledit porte-canule (3) comporte au moins une fente longitudinale primaire (38, 38') et au moins une fente longitudinale secondaire (39, 39') destinées à coopérer avec ledit taquet (20).

15. Fourreau (1) de protection selon l'une quelconque des revendications 1 à 14, **caractérisé en ce qu'**il présente une extrémité proximale (21) apte à recevoir la seringue, **en ce que** les moyens de transition secondaires (12) comportent un trou (16) apte à recevoir le porte-canule (3) une fois libéré des moyens de blocage provisoires lors d'un déplacement du porte-canule (3) vers l'extrémité proximale (21) du fourreau (1), et **en ce que** ledit déplacement longitudinal part du trou (16) vers une extrémité distale (22) du fourreau (1).

16. Ensemble d'injection (4) constitué par un fourreau (1) selon l'une quelconque des revendications 1 à 15, dans lequel est introduit une aiguille d'injection ou canule (2) disposée sur un support porte-canule (3).

17. Ensemblc d'injection (4) selon la revendication 16, **caractérisé en ce que** ledit porte-canule (3) présente au moins deux bossages longitudinaux (30, 30') positionnés diamétralement opposés par rapport à l'axe dudit porte-canule (3).

18. Seringue comportant une aiguille d'injection ou canule (2), ladite seringue étant équipée d'un fourreau (1) selon l'une quelconque des revendications 1 à 15.

## Claims

1. Protective sheath (1) for an injection needle or cannula (2) arranged on a cannula-holding support (3) which can be fitted onto a syringe, said sheath comprising guide means (14) making it possible at least to move said cannula holder (3) from a completely retracted position R, in which said cannula (2) is completely inside said sheath (1), to an ejection position E in which said cannula (2) projects at least partially outside said sheath (1), and vice versa, said sheath (1) also comprising final locking means (9) enabling said cannula holder (3) to be permanently locked in a retracted post-use position RAU, and comprising secondary transition means (12) actuated by a longitudinal movement of the cannula holder (3) and enabling the cannula holder (3) to move from a retracted pre-use position RPU to a retracted use position RU, and preliminary locking means (6) enabling said cannula holder (3) to be temporarily locked in the retracted pre-use position RPU, **characterized in that** it comprises preliminary locking means (6) comprising a distal wall of a hole intended to receive bosses of the cannula holder (3), the wall being designed to longitudinally retain the bosses of the cannula holder (3) in such a way that a distal end of the syringe can be clipped into the cannula holder.

2. Protective sheath (1) according to Claim 1, **characterized in that** it comprises primary transition means (11) enabling the cannula holder (3) to be brought into the retracted pre-use position RPU.

3. Protective sheath (1) according to Claim 1 or 2, **characterized in that** it has a proximal end (21) designed to receive the syringe, and **in that** the locking means (6) comprise a distal longitudinal stop.

4. Protective sheath (1) according to any one of Claims 1 to 3, **characterized in that** it comprises tertiary transition means (13) enabling the cannula holder (3) to be brought from a retracted use position RU to a retracted post-use position RAU.

5. Protective sheath (1) according to any one of Claims 2 to 4, **characterized in that** said primary (11), secondary (12) and tertiary (13) transition means are single-transition means.

6. Protective sheath (1) according to any one of Claims 2 to 5, **characterized in that** said primary transition means (11) and/or said secondary transition means (12) and/or said tertiary transition means (13) are each formed at least by a transition portion having, in longitudinal section, a recess (15) made in the inner face of said sheath (1), said single or said plural transition means having, at each of its or of their ends, a hole (16, 16') and, between these holes (16, 16'), an inclined flat section (17) progressively taking the depth p of said recess (15) toward a zero, or even negative, value.

7. Protective sheath (1) according to Claim 6, **characterized in that** said transition portion additionally comprises, in longitudinal section, a straight flat section (18, 18') between the inclined flat section (17) and the hole (16, 16').

8. Protective sheath (1) according to Claim 7, **characterized in that** said hole (16, 16') situated in the vicinity of the zero, or even negative, depth p is extended at least partially on each side of the inclined plane (17) by longitudinal slots (23, 23').

9. Protective sheath (1) according to any one of Claims 2 to 5, **characterized in that** said primary (11) and/or secondary (12) and/or tertiary (13) transition means are each formed by a transition portion formed by a lug.

10. Protective sheath (1) according to any one of Claims 1 to 9, **characterized in that** it comprises at its proximal end receiving means (10) enabling said cannula holder (3) to be positioned in an introduction position I.

11. Protective sheath (1) according to any one of Claims 1 to 10, **characterized in that** said receiving means (10) and/or said guide means (14) are formed at least by a longitudinally slit portion opening at least onto the internal face (23) of said sheath (1).

12. Protective sheath (1) according to any one of Claims 2 to 11, **characterized in that** said primary transition means (11) and/or said secondary transition means (12) and/or said tertiary transition means (13) and/or said guide means (14) are formed by two identical portions positioned diametrically opposite one another about an axis X of said sheath (1).

13. Protective sheath (1) according to any one of Claims 1 to 12, **characterized in that** said preliminary locking means (6) are formed by a lug (19).

14. Protective sheath (1) according to any one of Claims 1 to 13, **characterized in that** it comprises a pawl (20) extending toward the inside of said sheath (1), and **in that** said cannula holder (3) comprises at least one primary longitudinal slot (38, 38') and at least one secondary longitudinal slot (39, 39') which are intended to cooperate with said pawl (20).

15. Protective sheath (1) according to any one of Claims 1 to 14, **characterized in that** it has a proximal end (21) designed to receive the syringe, **in that** the secondary transition means (12) have a hole (16) designed to receive the cannula holder (3) once released from the temporary locking means during a movement of the cannula holder (3) toward the proximal end (21) of the sheath (1), and **in that** said longitudinal movement starts from the hole (16) toward a distal end (22) of the sheath (1).

16. Injection unit (4) formed by a sheath (1) according to any one of Claims 1 to 15, into which an injection needle or cannula (2) arranged on a cannula-holding support (3) is introduced.

17. Injection unit (4) according to Claim 16, **characterized in that** said cannula holder (3) has at least two longitudinal bosses (30, 30') positioned diametrically opposite one another with respect to the axis of said cannula holder (3).

18. Syringe comprising an injection needle or cannula (2), said syringe being equipped with a sheath (1) according to any one of Claims 1 to 15.

## Patentansprüche

1. Schutzhülse (1) für eine Injektionsnadel oder eine Kanüle (2), die an einem Kanülenträger (3) angeordnet ist, der an eine Spritze anpassbar ist, wobei die Hülle Führungsmittel (14) enthält, die wenigstens ermöglichen, dass sich der Kanülenträger (3) aus einer vollständig zurückgezogenen Position R, in der sich die Kanüle (2) vollständig innerhalb der Hülle (1) befindet, in eine Ausstoßposition E, in der die Kanüle (2) wenigstens teilweise aus der Hülle (1) vorsteht, und umgekehrt bewegen kann, wobei die Hülle (1) außerdem Endblockiermittel (9), die ermöglichen, den Kanülenträger (3) in einer zurückgezogenen Position RAU nach der Verwendung endgültig zu blockieren, sekundäre Übergangsmittel (12), die durch eine longitudinale Verlagerung des Kanülenträgers (3) betätigt werden und die Bewegung des Kanülenträgers (3) aus einer zurückgezogenen Position RPU vor der Verwendung in eine zurückgezogene Position RU während der Verwendung ermöglichen, und Mittel (6) zum vorläufigen Blockieren, die ein vorläufiges Blockieren des Kanülenträgers (3) in der zurückgezogenen Position RPU vor der Verwendung ermöglichen, enthält, **dadurch gekennzeichnet, dass** die Mittel (6) zum vorläufigen Blockieren eine distale Wand eines Lochs aufweisen, das dazu bestimmt ist, Höcker des Kanülenträgers (3) aufzunehmen, wobei die Wand die Höcker des Kanülenträgers (3) longitudinal in der Weise zurückhalten kann, dass ein distales Ende der Spritze in den Kanülenträger einrastbar ist.

2. Schutzhülle (1) nach Anspruch 1, **dadurch gekennzeichnet, dass** sie primäre Übergangsmittel (11) enthält, die ermöglichen, den Kanülenträger (3) in die zurückgezogene Position RPU vor der Verwendung zu bringen.

3. Schutzhülle (1) nach Anspruch 1 oder 2, **dadurch gekennzeichnet, dass** sie ein proximales Ende (21) aufweist, das die Spritze aufnehmen kann, und dass die Blockiermittel (6) einen distalen longitudinalen Anschlag aufweisen.

4. Schutzhülle (1) nach einem der Ansprüche 1 bis 3, **dadurch gekennzeichnet, dass** sie tertiäre Übergangsmittel (13) aufweist, die ermöglichen, den Kanülenträger (3) aus einer zurückgezogenen Position RU während der Verwendung in eine zurückgezogene Position RAU nach der Verwendung zu bringen.

5. Schutzhülle (1) nach einem der Ansprüche 2 bis 4, **dadurch gekennzeichnet, dass** die primären Übergangsmittel (11), die sekundären Übergangsmittel (12) und die tertiären Übergangsmittel (13) einen einzigen Übergang schaffen.

6. Schützhülle (1) nach einem der Ansprüche 2 bis 5, **dadurch gekennzeichnet, dass** die primären Übergangsmittel (11) und/oder die sekundären Übergangsmittel (12) und/oder die tertiären Übergangsmittel (13) jeweils aus wenigstens einem Übergangsteil gebildet sind, der im Längsschnitt eine in der Innenfläche der Hülle (1) verwirklichte Aussparung (15) aufweist, wobei die Übergangsmittel an jedem ihrer Enden ein Loch (16, 16') und zwischen diesen Löchern (16, 16') eine geneigte Fläche (17) aufweisen, die die Tiefe p der Aussparung (15) allmählich auf einen Wert Null oder sogar auf einen negativen Wert bringt.

7. Schutzhülle (1) nach Anspruch 6, **dadurch gekennzeichnet, dass** der Übergangsteil außerdem im Längsschnitt zwischen der geneigten Fläche (17) und dem Loch (16, 16') eine gerade Fläche (18, 18') aufweist.

8. Schutzhülle (1) nach Anspruch 7, **dadurch gekennzeichnet, dass** das Loch (16, 16'), das sich in der Nähe der Tiefe p befindet, die den Wert Null oder sogar einen negativen Wert hat, wenigstens teilweise auf jeder Seite der geneigten Ebene (17) durch Längsschlitze (23, 23') verlängert ist.

9. Schutzhülle (1) nach einem der Ansprüche 2 bis 5, **dadurch gekennzeichnet, dass** die primären Übergangsmittel (11) und/oder die sekundären Übergangsmittel (12) und/oder die tertiären Übergangsmittel (13) jeweils aus einem Übergangsteil gebildet sind, der aus einer Nase gebildet ist.

10. Schutzhülle (1) nach einem der Ansprüche 1 bis 9, **dadurch gekennzeichnet, dass** sie an ihrem proximalen Ende Aufnahmemittel (10) aufweist, die ermöglichen, den Kanülenträger (3) in einer Einführungsposition I zu positionieren.

11. Schutzhülle (1) nach einem der Ansprüche 1 bis 10, **dadurch gekennzeichnet, dass** die Aufnahmemittel (10) und/oder die Führungsmittel (14) wenigstens aus einem longitudinal geschlitzten Teil gebildet sind, der zumindest in die Innenfläche (23) der Hülle (1) mündet.

12. Schutzhülle (1) nach einem der Ansprüche 2 bis 11, **dadurch gekennzeichnet, dass** die primären Übergangsmittel (11) und/oder die sekundären Übergangsmittel (12) und/oder die tertiären Übergangsmittel (13) und/oder die Führungsmittel (14) aus zwei gleichen Teilen gebildet sind, die längs der Achse X der Hülle (1) diametral entgegengesetzt positioniert sind.

13. Schutzhülle (1) nach einem der Ansprüche 1 bis 12, **dadurch gekennzeichnet, dass** die vorläufigen Blockiermittel (6) durch eine Nase (19) gebildet sind.

14. Schutzhülle (1) nach einem der Ansprüche 1 bis 13, **dadurch gekennzeichnet, dass** sie einen Vorsprung (20) aufweist, der sich in die Hülle (1) erstreckt, und dass der Kanülenträger (3) wenigstens einen primären Längsschlitz (38, 38') und wenigstens einen sekundären Längsschlitz (39, 39') enthält, die dazu vorgesehen sind, mit dem Vorsprung (20) zusammenzuwirken.

15. Schutzhülle (1) nach einem der Ansprüche 1 bis 14, **dadurch gekennzeichnet, dass** sie ein proximales Ende (21) aufweist, das die Spritze aufnehmen kann, dass die sekundären Übergangsmittel (12) ein Loch (16) aufweisen, das den Kanülenträger (3) aufnehmen kann, sobald er von den vorläufigen Blockiermitteln bei einer Verlagerung des Kanülenträgers (3) zum proximalen Ende (21) der Hülle (1) freigegeben worden ist, und dass die longitudinale Verlagerung von dem Loch (16) zu einem distalen Ende (22) der Hülle (1) erfolgt.

16. Injektionsanordnung (4), die durch eine Hülle (1) nach einem der Ansprüche 1 bis 15 gebildet ist, in die eine Injektionsnadel oder Kanüle (2) eingeführt ist, die auf einem Kanülenträger (3) angeordnet ist.

17. Injektionsanordnung (4) nach Anspruch 16, **dadurch gekennzeichnet, dass** der Kanülenträger (3) wenigstens zwei longitudinale Höcker (30, 30') aufweist, die in Bezug auf die Achse des Kanülenträgers (3) diametral entgegengesetzt positioniert sind.

18. Spritze, die eine Injektionsnadel oder Kanüle (2) enthält, wobei die Spritze mit einer Hülle (1) nach einem der Ansprüche 1 bis 15 ausgerüstet ist.
